# EUROPEAN PATENT APPLICATION

(11) **EP 4 670 782 A1**
(43) Date of publication of application: **31.12.2025**
(21) Application number: 24759558.0
(22) Date of filing: 06.02.2024
(51) Int. Cl.: A61N 1/375

(54) **COMMUNICATION METHOD, SYSTEM, ELECTRONIC DEVICE, AND STORAGE MEDIUM**

(30) Priority: 24.02.2023 CN 202310164348
(71) Applicant: MicroPort Soaring CRM (Shanghai) Co., Ltd., Shanghai 201203 (CN)
(72) Inventor: ZHANG, Jianfeng, Shanghai 201203 (CN); YU, Peng, Shanghai 201203 (CN)
(74) Representative: Lavoix
(86) International application number: PCT/CN2024/076203
(87) International publication number: WO 2024/174881

(57) **Abstract**

The present invention allows communication among multiple devices in an implantable leadless pacing system by providing communication methods, an implantable leadless pacing system, an electronic device and a storage medium. The communication methods implemented on an implantable leadless pacemaker includes: based on an acquired intracavitary electrocardiogram (IC-ECG) signal, obtaining, by the implantable leadless pacemaker, a pacing event corresponding to the IC-ECG signal, and if the pacing event is a pacing event corresponding to the implantable leadless pacemaker, opening a first receive window for the implantable leadless pacemaker, waiting until broadcast information of any other device is not received within an opening duration of the latest first receive window and after completion of the pacing event, broadcasting first broadcast information by the implantable leadless pacemaker. This communication method can avoid any signal collision when used for communication among multiple devices in an implantable leadless pacing system over a single communication channel.

## Description

### TECHNICAL FIELD

The present invention relates to the field of medical devices and, in particular, to communication methods, an implantable leadless pacing system, an electronic device and a storage medium.

### BACKGROUND

All conventional implantable cardiac pacemakers are capable of duplex communications with the outside world, typically through wireless telemetry, for example, using electromagnetic induction coils, radio frequency (RF) wireless technology, Bluetooth technology, etc. However, such duplex communication approaches for conventional implantable cardiac pacemakers are barely applicable to implantable leadless pacemakers due to a small size, a limited battery capacity and other limitations of the latter type of pacemaker. According to public information, human body communication (HBC) seems a plausible communication approach for implantable leadless pacemakers. Specifically, an implantable leadless pacemaker and associated external unit (e.g., a programmer) may each have a built-in electrical signal modulator/demodulator (modem), and the external unit may be attached to the human body in a form like an electrocardiogram (ECG) patch and communicate with the implantable leadless pacemaker through tissue in the human body.

Conventional implantable cardiac resynchronization pacemakers are usually intended for right atrial, right ventricular and left ventricular pacing, which requires the implantation of multiple such implantable leadless pacemakers at different locations in the heart. In this case, in addition to communication among these implantable leadless pacemakers, it is also necessary for the implantable leadless pacemakers to communicate with an external unit (e.g., a programmer). Further, when the service life of any of the implantable leadless pacemakers expires, replacement with a new implantable leadless pacemaker would be necessary. In the course of replacement, however, there may be multiple implantable leadless pacemakers at the same location in the heart. For this reason, it is necessary to prevent signal collisions for duplex HBC among multiple devices. Conventionally, such multi-device communication is achieved, for example, by the use of carriers in different frequency bands. However, this approach will significantly increase the complexity of the involved implantable leadless pacemakers and could not provide the desired miniaturization of them.

It should be noted that the information disclosed in this Background section is merely intended to provide a better understanding of the general context of the present invention and should not be taken as an acknowledgement or any form of admission that the information forms part of the common general knowledge of those skilled in the art.

### SUMMARY OF THE INVENTION

It is an object of the present invention to avoid any signal collision in communication among multiple devices in an implantable leadless pacing system over a single communication channel while allowing implantable leadless pacemaker(s) not to consume more power, be miniaturized and significantly extend its/their service life, through presenting communication methods, an implantable leadless pacing system, an electronic device and a storage medium.

To this end, in one aspect of the present invention, there is provided a communication method used for communication among a plurality of devices in an implantable leadless pacing system. The plurality of devices includes at least two implantable leadless pacemakers, or at least one leadless pacemaker and at least one programmer. The communication method is implemented by each implantable leadless pacemaker and comprises:
based on an acquired intracavitary electrocardiogram (IC-ECG) signal, obtaining, by the implantable leadless pacemaker, a pacing event corresponding to the IC-ECG signal;
if the pacing event is a pacing event corresponding to the implantable leadless pacemaker, opening a first receive window of the implantable leadless pacemaker;
waiting until broadcast information of any other device is not received within an opening duration of the latest first receive window and after completion of the pacing event, broadcasting first broadcast information by the implantable leadless pacemaker, wherein the first broadcast information contains identification information of the implantable leadless pacemaker and the pacing event.

Optionally, the communication method may further comprise:
opening the first receive window for the implantable leadless pacemaker at the beginning of each cardiac cycle.

After the pacing event is determined to be a pacing event corresponding to the implantable leadless pacemaker, the communication method may further comprise:
if a type of the pacing event is determined to be an abnormal pacing event, delivering a pacing pulse by the implantable leadless pacemaker to complete the pacing event.

Optionally, the communication method may further comprise:
with a first predetermined period being taken as a cycle, after completion of all pacing events in at least one cardiac cycle, broadcasting second broadcast information by the implantable leadless pacemaker through:
opening a second receive window by the implantable leadless pacemaker;
waiting until broadcast information of any other device is not received within an opening duration of the latest second receive window, broadcasting the second broadcast information by the implantable leadless pacemaker,
wherein the second broadcast information contains the identification information of the implantable leadless pacemaker, and the first predetermined period comprises a first number of cardiac cycles, where the first number is an integer greater than or equal to 1.

Optionally, the communication method may further comprise:
after each implantable leadless pacemaker broadcasts the second broadcast information, opening a third receive window for its reception of third broadcast information from the programmer, wherein the third broadcast information contains programming information from the programmer for the implantable leadless pacemaker.

Optionally, opening duration(s) of the first receive window, the second receive window and/or the third receive window of the implantable leadless pacemaker may be pre-configured according to a location where the implantable leadless pacemaker is implanted, or set through the programming information of the programmer.

To the above end, the present invention provides another communication method used for communication among a plurality of devices in an implantable leadless pacing system. The plurality of devices includes at least two implantable leadless pacemakers, or at least one leadless pacemaker and at least one programmer. The communication method is implemented by each programmer and comprises:
opening, by a programmer, a fourth receive window before it broadcasts third broadcast information; and
waiting until broadcast information of any other device is not received within an opening duration of the latest fourth receive window and then broadcasting the third broadcast information.

Optionally, waiting until broadcast information of any other device is not received within the opening duration of the latest fourth receive window and then broadcasting the third broadcast information may further comprise:
broadcasting the third broadcast information by the programmer after it receives second broadcast information from the implantable leadless pacemaker, wherein the third broadcast information further contains identification information of the implantable leadless pacemaker associated with the programming information.

Optionally, the communication method may further comprise:
determining a broadcast duration of each implantable leadless pacemaker from both a time interval for broadcasting of the second broadcast information by the implantable leadless pacemaker and a channel occupancy duration of the broadcast information;
obtaining a maximum broadcast duration from the broadcast duration of each implantable leadless pacemaker; and
determining a duration of the fourth receive window by:
   taking a sum of durations of a second predetermined period, a maximum broadcast interval of the second broadcast information of the implantable leadless pacemaker(s) and a broadcast time of the second broadcast information of the implantable leadless pacemaker as the opening duration of the fourth receive window, wherein the second predetermined period comprises a second number of cardiac cycles, which is an integer greater than or equal to 1; wherein the maximum broadcast interval of the second broadcast information of the implantable leadless pacemaker(s) is a time interval between a beginning time of transmission of the first one of second broadcast information and an beginning time of transmission of the last one of second broadcast information; and wherein the broadcast time of the second broadcast information of the implantable leadless pacemaker is a channel occupancy duration of the last one of the second broadcast information.

To the above end, the present invention also provides an implantable leadless pacing system comprising at least two implantable leadless pacemakers, or at least one leadless pacemaker and at least one programmer, wherein each implantable leadless pacemaker implements any variant of the first communication method as defined above to perform data transmission with the other implantable leadless pacemaker(s) and/or the programmer, and each programmer implements any variant of the second communication method as defined above to perform data transmission with the implantable leadless pacemaker.

To the above end, the present invention also provides an electronic device comprising a processor and a memory device, the memory device storing thereon a computer program, which, when executed by the processor, implements one of the communication methods as defined above.

To the above end, the present invention also provides a readable storage medium storing thereon a computer program, which, when executed by a processor, implements one of the communication methods as defined above.

The communication methods, implantable leadless pacing system, electronic device and storage medium of the present invention have the following advantages over the prior art.

The communication methods are provided to achieve communication among multiple devices in an implantable leadless pacing system, which include at least two implantable leadless pacemakers, or at least one leadless pacemaker and at least one programmer. As can be readily seen from the above description, both the communication method implemented by each implantable leadless pacemaker and the communication method implemented by each programmer follow the following basic principles: before any device transmits data, a receive window is opened for it; and subsequently, it waits until broadcast information of any other device is not received within opening duration of the latest receive window, and then transmits the data and broadcasts its broadcast information. Thus, the communication methods of the present invention essentially entail a communication protocol, which allows for signal collision-free communication among multiple devices in an implantable leadless pacing system over a single communication channel. In the communication methods provided by the present invention is based on a feature of multi-device and single channel, by utilizing the heartbeat cycle to achieve the communication among multiple devices in an implantable leadless pacing system, and configuring reception windows to enable multiple devices to communicate during their appropriate time intervals to prevent signal collisions. In this way, communication of carriers at the same frequency among multiple devices in the same patient's body over the same communication channel can be achieved without increasing the complexity of an implantable leadless pacemaker design used. In addition, in contrast to the conventional practice of using carriers in different frequency bands, which leads to high power consumption of implantable leadless pacemakers involved, this can significantly extend the service life of the implantable leadless pacemakers, thereby saving resources and reducing the adverse influence on the patient's body of otherwise necessary frequent replacement of the implantable leadless pacemakers. Furthermore, the communication methods are directly applicable to existing implantable leadless pacing systems without making any hardware modifications to their implantable leadless pacemakers or programmers, making them easy-to-implement and low-cost methods.

The implantable leadless pacing system, electronic device and storage medium of the present invention are based on the same inventive concept as the communication methods of the present invention and therefore have at least all the advantages thereof. Further detailed description of those advantages is not repeated herein for the sake of brevity.

### BRIEF DESCRIPTION OF THE DRAWINGS

Fig. 1 schematically illustrates a connection between a conventional implantable leadless pacing system and a patient's body.
Fig. 2 is a schematic overall flowchart of a communication method according to a first embodiment of the present invention, which is implemented by an implantable leadless pacemaker.
Fig. 3 shows a specific example of an implantable leadless pacing system implementing a communication method according to first and second embodiments of the present invention.
Fig. 4 is a schematic overall flowchart of a communication method according to a second embodiment of the present invention, which is implemented by a programmer.
Fig. 5 is a schematic diagram showing the structure of an implantable leadless pacing system according to a third embodiment of the present invention.
Fig. 6 is a block diagram schematically showing the structure of an electronic device according to an implementation of the fourth embodiment of the present invention.

List of Reference Numerals
programmer-111; body surface ECG patches -121, 122, 123; implantable leadless pacemaker- Ca, Cb, Cc; heart-130;
heartbeat event-140; first receive window-151a, 151b, 151c; second receive window-152a, 152b, 152c; third receive window-153a, 153b, 153c; fourth receive window-154; transmit window-161a, 161b, 161c, 162a, 162b, 162c, 163; broadcast window-170;
second number of cardiac cycles-T1; maximum broadcast interval-T2; broadcast time-T3;
processor-210; communication interface-220; memory device-230; communication bus-240.

### DETAILED DESCRIPTION

Communication methods, implantable leadless pacing systems, electronic devices and storage media proposed herein will be described in greater detail below with reference to the accompanying drawings. From the following description, advantages and features of the present invention will be more apparent. Note that the figures are provided in a very simplified form not necessarily drawn to exact scale for the only purpose of helping to explain the disclosed embodiments in a more convenient and clearer way. In order that the objects, features and advantages of the present invention can be more apparent and readily understood, reference is to be made to the accompanying drawings. It would be recognized that architectural, proportional, dimensional and other details in the figures are presented only for the purpose of facilitating, in conjunction with the disclosure herein, the understanding and reading of those familiar with the art, rather than being intended to limit conditions under which the present invention can be implemented. Therefore, any and all architectural modifications, proportional variations or dimensional changes that achieve the same or similar benefits and objects as the present invention are considered to fall within the scope of the teachings herein. The specific design features of the invention as disclosed herein, including, for example, specific dimensions, orientations, locations and shapes, will be determined in part by the particular intended application and use environment. Additionally, in the embodiments described below, same reference numerals may be sometimes used to refer to the same or functionally similar elements throughout different figures, while description thereof may not be repeated. In this specification, same reference numerals and letters refer to same items in the figures, and thus once an item is defined in one figure, it may not be discussed for following figures. Further, if a method is described herein as comprising a series of steps, the order of these steps as presented herein is not necessarily the only order in which they can be performed, and some of the stated steps may be omitted and/or other steps not described herein may be added to the method.

It is noted that, as used herein, relational terms such as first and second, etc., are only used to distinguish one entity or operation from another entity or operation, and do not necessarily require or imply these entities having such an order or sequence. Moreover, the terms "comprise," "include," or any other variations thereof are intended to cover a non-exclusive inclusion within a process, method, article, or apparatus that comprises a list of elements including not only those elements but also those that are not explicitly listed, or other elements that are inherent to such processes, methods, goods, or equipment. In the case of no more limitation, the element defined by the sentence "includes a ..." does not exclude the existence of another identical element in the process, the method, or the device including the element. As used herein, the singular forms "a", "an" and "the" include plural referents, and the term "or" is generally employed in the sense of "and/or", "a number of" is generally employed in the sense of "at least one", and "at least two" is generally employed in the sense of "two or more". Additionally, the use of the terms "first", "second" and "third" herein is intended for illustration only and is not to be construed as denoting or implying relative importance or as implicitly indicating the numerical number of the referenced items.

Before describing communication methods, implantable leadless pacing systems, electronic devices and storage media proposed herein in detail, the general architecture of conventional implantable leadless pacing systems will be briefed below, in order to facilitate an understanding of the present invention.

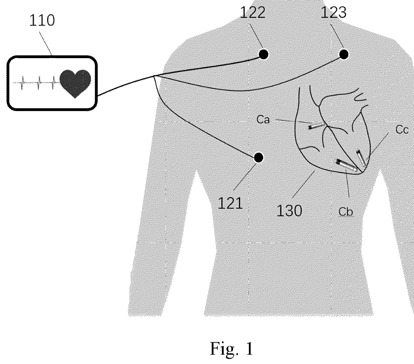

Conventional implantable leadless pacing systems typically include a programmer and an implantable leadless pacemaker. Specifically, reference is made to Fig. 1, which schematically illustrates a connection between a conventional implantable leadless pacing system and a patient's body. For ease of description, the implantable leadless pacing system shown in Fig. 1 includes a programmer 111 and three implantable leadless pacemakers Ca, Cb, Cc, for example. As can be seen from Fig. 1, the programmer 111 is coupled to the body through the body surface electrocardiogram (ECG) patches 121, 122, 123. The implantable leadless pacemakers Ca, Cb, Cc are implanted in associated chambers of the heart 130, for example, the implantable leadless pacemaker Ca is implanted in the right atrium, the implantable leadless pacemaker Cb is implanted in the right ventricle and the implantable leadless pacemaker Cc is implanted in the left ventricle. Since the three implantable leadless pacemakers Ca, Cb, Cc are all located in the same patient's body, and due to their size and power consumption limitations, they communicate with one another and with the programmer 111 via a same channel. For this reason, in order to achieve effective communication, it is necessary to prevent signals from interfering with one another. As noted above, although such duplex communication among multiple devices can be conventionally achieved by the use of carriers in different frequency bands, employing this approach would significantly complicate the implantable leadless pacemakers and could not provide the desired miniaturization of the implantable leadless pacemakers.

In view of this, it is a principal object of the present invention to provide communication methods for data transmission among multiple devices in an implantable leadless pacing system. The multiple devices preferably include at least two implantable leadless pacemakers, or include at least one leadless pacemaker and at least one programmer. These communication methods (which essentially entail a communication protocol for the implantable leadless pacing system) are based on the following operating principles: before any device transmits data, a receive window is opened for it; and subsequently, it waits until broadcast information of any other device is not received within opening duration of the most recent receive window, and then transmits the data and broadcasts its broadcast information. The communication method provided by the present invention is based on a feature of multi-device and single channel, by utilizing the heartbeat cycle to achieve the communication among multiple devices in an implantable leadless pacing system, and configuring reception windows to enable multiple devices to communicate during their appropriate time intervals to prevent signal collisions. In this way, communication of a carrier at the same frequency among multiple devices in the same patient's body over the same communication channel can be achieved without increasing the design complexity of an implantable leadless pacemaker. In addition, in contrast to the conventional practice of using carriers in different frequency bands, which leads to high power consumption of implantable leadless pacemakers involved, this can significantly extend the service life of the implantable leadless pacemakers, thereby saving resources and reducing the adverse influence on the patient's body of otherwise necessary frequent replacement of the implantable leadless pacemakers. Furthermore, the communication methods are directly applicable to existing implantable leadless pacing systems without making any hardware modifications to their implantable leadless pacemakers or programmers, making them easy-to-implement and low-cost methods.

In order to provide a better understanding of the present invention and to facilitate its description, a communication method according to a first embodiment is described below from the point of view of an implantable leadless pacemaker, and a communication method according to a second embodiment from the point of view of a programmer. Both the first and second embodiments are set forth in the context of use in an implantable leadless pacing system with a programmer and three implantable leadless pacemakers, as an example. It is particularly noted that, and as would be appreciated by those skilled in the art, the present invention is not limited to any number of implantable leadless pacemakers or any number of programmers in an implantable leadless pacing system, in which it is implemented. In some embodiments, it may be implemented in an implantable leadless pacing system with only one or more implantable leadless pacemakers. In some other embodiments, it may be implemented in an implantable leadless pacing system with one or more implantable leadless pacemakers and one or more programmers. In particular, in communication methods disclosed herein, carrier at the same frequency is used for communication throughout an implantable leadless pacing system. It also should be noted that the present invention is not limited to any particular modulation or demodulation method, and detailed description thereof will be omitted herein. For more detailed information about modulation and demodulation, reference can be made to literature about the frequency-shift keying (FSK) technique or variants thereof.

### Embodiment 1

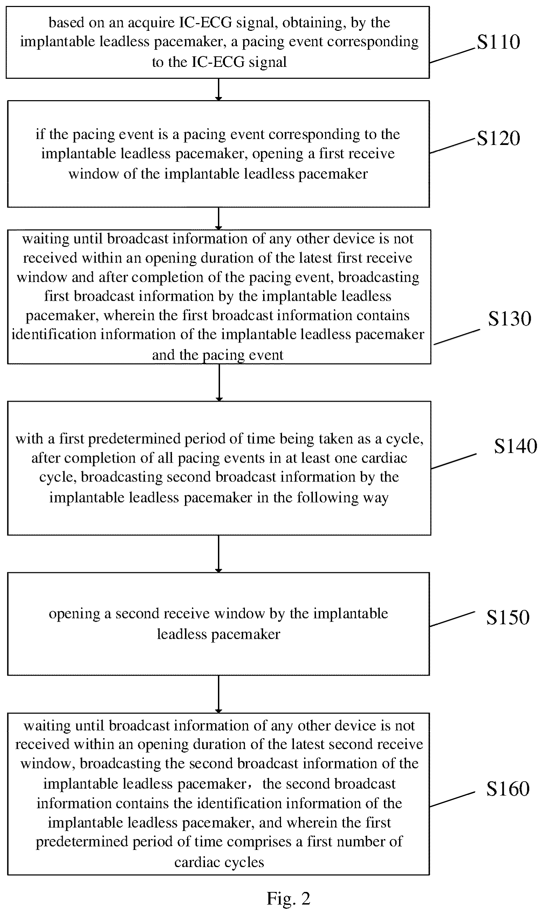

In a first embodiment of the present invention, there is provided a communication method for multiple devices in an implantable leadless pacing system. The multiple devices include at least two implantable leadless pacemakers, or include at least one leadless pacemaker and at least one programmer. The communication method is implemented by each implantable leadless pacemaker. Specifically, reference is made to Fig. 2, a schematic overall flowchart of the communication method of the present embodiment. As can be seen from Fig. 2, the communication method of the present embodiment includes the steps of:
S110: based on an acquired intracavitary electrocardiogram (IC-ECG) signal, obtaining, by the implantable leadless pacemaker, a pacing event corresponding to the IC-ECG signal;
S120: if the pacing event is a pacing event corresponding to the implantable leadless pacemaker, opening a first receive window for the implantable leadless pacemaker;
S130: waiting until broadcast information of any other device is not received within an opening duration of the latest first receive window and after completion of the pacing event, broadcasting first broadcast information by the implantable leadless pacemaker, wherein the first broadcast information contains identification information of the implantable leadless pacemaker and the pacing event.

Therefore, in the communication method of the present embodiment, each implantable leadless pacemaker first obtains, based on an acquired IC-ECG signal, a pacing event corresponding to the IC-ECG signal; then it determines whether the pacing event is a pacing event corresponding to itself; if so, opening a first reception window corresponding to the leadless implantable pacemaker; after waiting until broadcast information of any other device is not received within an opening duration of the most recent first receive window and after the pacing event is completed, the implantable leadless pacemaker broadcasts first broadcast information, which includes identification information of the implantable leadless pacemaker and the pacing event. In this way, each implantable leadless pacemaker transmits its broadcast information after opening at least one first receive window (equivalent to its waiting for a predetermined period of time). If broadcast information of any other device (i.e., another implantable leadless pacemaker or a programmer) is not received in an opening duration of the first receive window, it is determined that the communication channel is currently out of use and the broadcasting of the broadcast information that indicates that the implantable leadless pacemaker has completed the pacing event at this time will thereby not lead to a collision with any signal being transmitted on the communication channel, ensuring effective transmission of the broadcast information. In addition, the broadcast information broadcast from the implantable leadless pacemaker, on the one hand, facilitates other devices intending to occupy the channel to monitor during their respective reception windows, thereby avoiding signal collisions between the broadcast information from this implantable leadless pacemaker and broadcast information from other devices; on the other hand, facilitates the other device(s) in the implantable leadless pacing system to know whether the implantable leadless pacemaker from which the current broadcast information is broadcast has completed pacing event.

It should be noted that, and as would be appreciated by those skilled in the art, the present invention is not limited to any particular length of the first receive window. In one preferred implementation, for each implantable leadless pacemaker, the length of the first receive window may be appropriately configured according to the location where the pacemaker is implanted so that it is generally unnecessary for the pacemaker to wait for opening duration of multiple first receive windows. That is, only one receive window has been waited in step S130: waiting until broadcast information of any other device is not received within opening duration of the most recent first receive window and after completing the pacing event, thereby allowing for more efficiency data transmission.

In one exemplary implementation, each implantable leadless pacemaker opens a first receive window at the beginning of each cardiac cycle. In this way, the communication method provided in this embodiment (each implantable leadless pacemaker opens its respective first reception window at the beginning of each cardiac cycle), fully utilizes that within each cardiac cycle, the pacing event(s) of the implantable leadless pacemaker(s) is/are occurred in an order based on its/ their implanted location(s), thus, determining and obtaining the beginning of the cardiac cycle according to an acquired IC-ECG signal to open their respective first reception windows.

In particular, in step S120, when the pacing event is determined to be a pacing event corresponding to the implantable leadless pacemaker, the communication method may further include:
if a type of the pacing event is determined to be abnormal pacing, delivering a pacing pulse by the implantable leadless pacemaker to complete the pacing event.

In this way, when the pacing event corresponding to the IC-ECG signal is abnormal, the implantable leadless pacemaker delivers a pacing pulse to drive the endocardial myocardium to complete pacing (contraction), thereby ensuring a steady heartbeat and maintaining normal functioning of a patient' body. It should be noted that, and as would be appreciated, the pacing event indicated by the IC-ECG signal may be a spontaneous or abnormal pacing event. A spontaneous pacing event corresponds to a normal endomyocardial contraction (the pacing event is completed by the endocardial myocardium), which does not necessitate delivery of a pacing pulse by the implantable leadless pacemaker. That is, when the pacing event is determined to be spontaneous (normal) pacing, the implantable leadless pacemaker does nothing (i.e., does not deliver a pacing pulse).

An abnormal pacing event corresponds to a failure of normal endomyocardial contraction. When this happens, the implantable leadless pacemaker delivers a pacing pulse, which is transferred via an electrode of the implantable leadless pacemaker and stimulates a contraction of the endomyocardium that the electrode contacts. That is, the pacing event is completed with the aid of the implantable leadless pacemaker.

Additionally, the first broadcast information contains the identification information of the implantable leadless pacemaker and the pacing event, enabling the programmer(s) and the other implantable leadless pacemaker(s) to be aware of which cardiac chamber (e.g., atrium, ventricle) initiated the current pacing event and details of the pacing event, thereby better ensuring a steady heartbeat and maintaining normal functioning of a patient' body. For example, the implantable leadless pacing system may include a programmer, a right atrial pacemaker implanted in a patient's right atrium, a left ventricular pacemaker implanted in the patient's left ventricle and a right ventricular pacemaker implanted in the patient's right ventricle. When the right atrial pacemaker implanted in the patient's right atrium senses a normal right atrial pacing event at a certain instant of time, the identification information of the first broadcast information transmitted by the right atrial pacemaker is the right atrial pacemaker and the pacing event is spontaneous pacing. Then, from the identification information (the right atrial pacemaker) in the first broadcast information, the programmer, the left ventricular pacemaker and the right ventricular pacemaker can identify that pacing event has occurred in the right atrium. Additionally, from the broadcast event (spontaneous pacing), the programmer, the left atrial pacemaker and the right ventricular pacemaker can also identify that the pacing in the right atrium is spontaneous pacing, facilitating the left ventricular pacemaker and the right ventricular pacemaker in confirming their corresponding pacing event. Likewise, when the right atrial pacemaker implanted in patient's right atrium senses an abnormal pacing event in the right atrium, the identification information of the first broadcast information transmitted by the right atrial pacemaker is the right atrial pacemaker and the pacing event is the abnormal pacing. Then, from the identification information (the right atrial pacemaker) in the first broadcast information, the programmer, the left ventricular pacemaker and the right ventricular pacemaker can identify that pacing event has occurred in the right atrium. Additionally, from the broadcast event (the abnormal pacing event), the programmer, the left ventricular pacemaker and the right ventricular pacemaker can also identify that the pacing in the right atrium is abnormal, and the left ventricular pacemaker and the right ventricular pacemaker will complete their corresponding pacing time at appropriate latter timing to better maintain normal functioning of the patient' body.

The aforementioned pacing event may be either an atrial or ventricular pacing event, as is sensed actually.

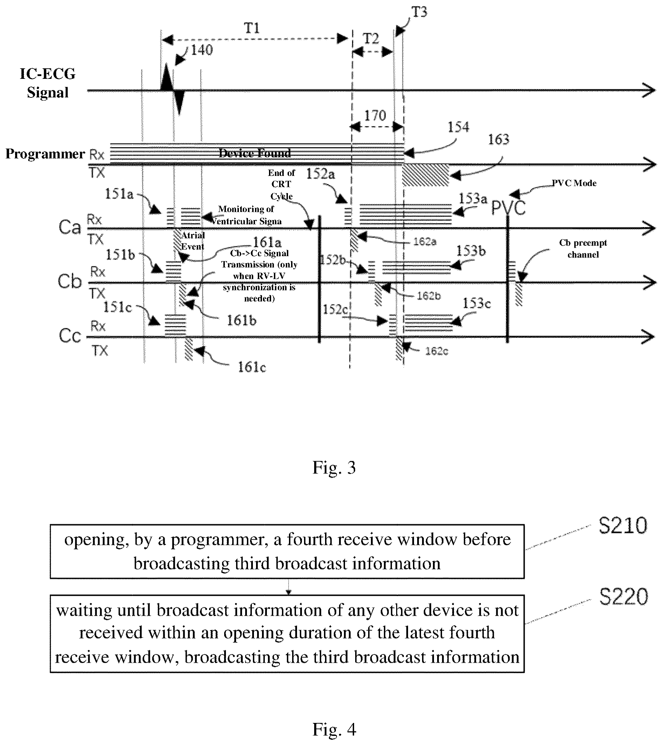

More particular reference is made to Figs. 1 and 3. Fig. 3 schematically illustrates a specific example of an implantable leadless pacing system implementing a communication method according to first and second embodiments of the present invention. As an example, as shown in Fig. 1, the implantable leadless pacing system includes a programmer 111 and three implantable leadless pacemakers Ca, Cb, Cc. The implantable leadless pacemaker Ca is implanted in a patient's right atrium for right atrial pacing. The implantable leadless pacemaker Cb is implanted in the patient's right ventricle for right ventricular pacing. The implantable leadless pacemaker Cc is implanted in the patient's left ventricle for left ventricular pacing. As would be appreciated by those skilled in the art, the present invention is not limited to any particular locations where the implantable leadless pacemakers are implanted, and the atrium/ventricle of each implantable leadless pacemaker and a pacing order may be programmed according to their implanted locations. In this specific example, it is programmed to set that the implantable leadless pacemaker Ca first paces the right atrium, then the implantable leadless pacemaker Cb paces the right ventricle, finally the implantable leadless pacemaker Cc paces the left ventricle. In Fig. 3, timing is indicated by horizontal arrows, and a heartbeat event 140 is indicated by black solid triangles. Moreover, each receive window is indicated by "Rx" and each broadcast (or transmit) window is indicated by "TX". Fig. 3 illustrates how the implantable leadless pacemakers communicate with each other and with the programmer in the implantable leadless pacing system implementing the communication method in this embodiment in a cardiac cycle.

As can be seen from Fig. 3, in this embodiment, at the beginning of the cardiac cycle, the implantable leadless pacemakers Ca, Cb, Cc open respective first receive windows. More specifically, as soon as the implantable leadless pacemaker Ca senses an atrial event according to an IC-ECG signal (e.g., an endocardial signal acquired from the endocardium), it opens a first receive window. Optionally, the open may occur at the beginning of each cardiac cycle. As shown in Fig. 3, the implantable leadless pacemakers Ca, Cb, Cc may open respective first receive windows 151a, 151b, 151c at a time around the middle of an earlier half of a peak in a wave of the heartbeat event 140 (indicated by the black solid triangles in Fig. 3). If the implantable leadless pacemaker Ca detects that there is no broadcast information from any other device on the current channel within the opening duration of its corresponding first receive window 151a, then after the completion of the atrial pacing event (i.e., corresponding pacing event), the implantable leadless pacemaker Ca may broadcast information (duration occupied by the broadcast information on the channel as shown by transmit window 161a of Ca, as shown in Fig. 3) indicating the completion of the atrial pacing event (spontaneous pacing or an assisted pacing by a pacing pulse delivered from the implantable leadless pacemaker Ca). Similarly, once sensing a right ventricular pacing event (i.e., a ventricular pacing event), the implantable leadless pacemaker Cb will pace the right ventricle if the sensed pacing event is abnormal. Then, following the completion of the broadcast information indicating the atrial pacing event by pacemaker Ca (as shown in Fig.3, the beginning of the transmit window 161b of Cb is after the completion of the transmit window 161a of Ca), the implantable leadless pacemaker Cb will broadcast broadcast information indicating the completion of the right ventricular pacing event (duration occupied by the broadcast information on the channel as shown by transmit window 161b of Cb, in Fig. 3). Once sensing a left ventricular pacing event (i.e., a ventricular pacing event), the implantable leadless pacemaker Cc will pace the left ventricle if the sensed pacing event is abnormal. Then, following the completion of the broadcast information indicating the right ventricular pacing event by pacemaker Cb (as shown in Fig.3, the beginning of the transmit window of Cc is after the completion of the transmit window 161b of Cb), the implantable leadless pacemaker Cc will broadcast broadcast information indicating the completion of the left ventricular pacing (duration occupied by the broadcast information on the channel as shown by transmit window 161c of Cc).

It should be noted that, and as would be appreciated by those skilled in the art, apart from the heartbeat event 140 (indicated by the black solid triangles in Fig. 3) and a cardiac resynchronization therapy (CRT) cycle (a pacing cycle of the implantable leadless pacing system, as shown in Fig. 3), the cardiac cycle also includes an interval (i.e., a time interval from the end of the pacing cycle of the implantable leadless pacing system in the previous cardiac cycle to the beginning of the heartbeat event in the next cardiac cycle). Accordingly, continued reference is made to Figs. 2 and 3. As can be seen from Fig. 2, in this embodiment, the communication method further includes the step of:
S140: with a first predetermined period being taken as a cycle, after the completion of all pacing events in at least one cardiac cycle, broadcasting second broadcast information by each implantable leadless pacemaker through:
S150: opening a corresponding second receive window for the implantable leadless pacemaker; and
S160: waiting until broadcast information of any other device is not received within an opening duration of the most recent second receive window, broadcasting the second broadcast information by the implantable leadless pacemaker.

The second broadcast information contains identification information of the implantable leadless pacemaker, and the first predetermined period comprises a first number of cardiac cycles. The first number is integer greater than or equal to 1.

Thus, in the communication method of the present embodiment, each implantable leadless pacemaker can transmit second broadcast information, which contains its identification information (e.g., a unique ID of the implantable leadless pacemaker) within an interval between heartbeat events and allows the programmer to identify the implantable leadless pacemaker. Further, since each implantable leadless pacemaker opens a respective second receive window before it transmits the second broadcast information, any signal collision of second broadcast information from respective implantable leadless pacemakers can be prevented.

It should be noted that, and as would be appreciated by those skilled in the art, the first predetermined period may preferably be multiple cardiac cycles. That is, it may be not necessary for the implantable leadless pacemakers to transmit within the interval of a single cardiac cycle, but may transmit after multiple cardiac cycles. The first number may be equal to one or more than one (multiple), and may be modified via the programmer. In the case of the first predetermined period being multiple cardiac cycles, the implantable leadless pacemakers will consume even less power, and their service life can be therefore further extended.

More particular reference is made to Fig. 3. As shown, the channel duration occupied by the second broadcast information broadcast from the implantable leadless pacemaker Ca corresponds to a duration of a transmit window 162a of the implantable leadless pacemaker Ca. The channel duration occupied by the second broadcast information broadcast from the implantable leadless pacemaker Cb corresponds to a duration of a transmit window 162b of the implantable leadless pacemaker Cb. The channel duration occupied by the second broadcast information broadcast from the implantable leadless pacemaker Cc corresponds to a duration of a transmit window 162c of the implantable leadless pacemaker Cc. A total length of the second broadcast information broadcast from the implantable leadless pacemakers Ca, Cb, Cc is a broadcast window 170, as shown in Fig. 3. As can also be seen from Fig. 3, the second receive windows 152a, 152b, 152c are opened by the respective implantable leadless pacemakers before they transmit the second broadcast information. Taking the implantable leadless pacemaker Ca as an example, its opened second reception window corresponds to the second reception window 152a between the dashed vertical line preceding Ca's broadcast and the end of the CRT cycle in Fig. 3, and the opening of the second receive window 152b for the implantable leadless pacemaker Cb as following the completion of the second broadcast information by the implantable leadless pacemaker Ca.

More particularly, in an exemplary implementation, the communication method further includes:
for each implantable leadless pacemaker, after it broadcasts the second broadcast information, opening a third receive window for reception of third broadcast information from the programmer, the third broadcast information contains programming information from the programmer to the implantable leadless pacemaker.

Thus, the communication method of the present embodiment can not only ensure that broadcast information from any implantable leadless pacemaker will not collide with broadcast information from another implantable leadless pacemaker, but can also ensure, through opening the third receive windows for the respective implantable leadless pacemakers after they broadcast the second broadcast information, that the implantable leadless pacemakers are able to receive the third broadcast information from the programmer. Specifically, with continued reference to Fig. 3, a third receive window 153a is opened for the implantable leadless pacemaker Ca after it transmits the second broadcast information. Similarly, a third receive window 153b is opened for the implantable leadless pacemaker Cb after it transmits the second broadcast information, and a third receive window 153c is opened for the implantable leadless pacemaker Cc after it transmits the second broadcast information.

For example, after receiving the second broadcast information (162a, 162b, 162c of Fig. 3 in this embodiment) from all the implantable leadless pacemakers, the programmer may transmit data specifying which one of the implantable leadless pacemakers is to be programmed, i.e., which one of the implantable leadless pacemakers is the destination of the transmitted data (these implantable leadless pacemakers are individually programmable), and the specified implantable leadless pacemaker may respond.

Preferably, length(s) of the first receive window and/or the second receive window and/or the third receive window of the implantable leadless pacemaker is(are) pre-configured according to a location where the implantable leadless pacemaker is implanted, or set through the programming information of the programmer.

With continued reference to Fig. 3, in a premature ventricular contraction (PVC) mode, the implantable leadless pacemakers Ca, Cb, Cc may not follow the order discussed above, and the implantable leadless pacemaker Cb or Cc may preempt the communication channel to transmit a ventricular pacing event. Even in this case, since implantable leadless pacemakers Ca, Cb, Cc open the first receive windows 151a, 151b, 151c to allow them to monitor whether is on-going data transmission on the communication channel and transfer data via the communication channel when there is no data being currently transmitted thereon. As shown in Fig. 3, under PVC mode, the implantable leadless pacemaker Cb can successfully preempt the communication channel without causing signal collisions.

### Embodiment 2

In a second embodiment, there is provided a method for communication among multiple devices in an implantable leadless pacing system. The multiple devices include at least two implantable leadless pacemakers, or include at least one leadless pacemaker and at least one programmer. In contrast to the first embodiment, the communication method of this embodiment is implemented by each programmer. Specifically, reference is made to Fig. 4. As can be seen from Fig. 4, the communication method of the present embodiment includes the steps of:
S210: opening a fourth receive window by the programmer before it broadcasts third broadcast information; and
S220: waiting until broadcast information of any other device is not received within opening duration of the most recent fourth receive window and then broadcasting the third broadcast information.

Thus, in the communication method of the present embodiment, before broadcasting third broadcast information, the programmer opens a fourth receive window and then waits until broadcast information of any other device is not received within opening duration of the most recent fourth receive window, thereby preventing the third broadcast information from the programmer from colliding with broadcast information from an implantable leadless pacemaker or from another programmer, which is being currently transmitted on the communication channel.

Specifically, continuing the example of Fig. 3, the fourth receive window 154 for each programmer is labeled as "Device Found" and the channel duration occupied by the third broadcast information corresponds to a transmit window 163 of the programmer. As can be readily found from the transmit window 163 for the programmer and the channel duration occupied by the message broadcast for the respective implantable leadless pacemakers Ca, Cb, Cc shown in Fig. 3, the programmer transmits the third broadcast information without on-going data transmission on the communication channel (as shown in Fig. 3, the third broadcast information is transmitted by the programmer after transmission of the second broadcast messages by the respective implantable leadless pacemakers Ca, Cb, Cc is completed).

Specifically, in one exemplary implementation, waiting until broadcast information of any other device is not received within opening duration of the most recent fourth receive window and then broadcasting the third broadcast information may include:
broadcasting the third broadcast information by the programmer after it receives second broadcast information from the implantable leadless pacemaker, wherein the third broadcast information contains identification information of implantable leadless pacemaker associated with the programming information. With this arrangement, the communication method of this present invention can effectively ensure smooth data transmission between the programmer and the implantable leadless pacemaker.

More specifically, according to a preferred implementation, in the communication method, a length of the fourth receive window is determined as follows.

The sum of lengths of a second predetermined period, a maximum broadcast interval of the second broadcast information of the implantable leadless pacemaker(s) and a broadcast time of the second broadcast information of implantable leadless pacemaker is taken as the length of the fourth receive window. The second predetermined period includes a second number of cardiac cycles. The second number is an integer greater than or equal to 1. The maximum broadcast interval of the second broadcast information of the implantable leadless pacemaker(s) is a time interval between the beginning time of transmission of the first one of second broadcast information and the beginning time of transmission of the last one of second broadcast information. The broadcast time of the second broadcast information of the implantable leadless pacemaker is equal to a period in which the communication channel is occupied by the last one of second broadcast information.

With this arrangement, through appropriately configuring the fourth receive window for programmer, the communication method of the present invention can avoid any signal collision and additionally reduce power consumption of the programmer(s) and thereby extend the service life thereof while not adversely affecting data transmission between the programmer and the implantable leadless pacemaker.

Specifically, continuing the example of Fig. 3, in a preferred implementation, in response to a need for programming by the programmer 111, the programmer 111 may enter the "Device Identification" mode, and a fourth receive window 154 may be opened to allow it to monitor broadcast signals from the implantable leadless pacemakers Ca, Cb, Cc. In order to prevent any signal collision and enable the monitoring of broadcast information from all the implantable leadless pacemakers Ca, Cb, Cc, the opened monitoring time is desirably equal to the second number of cardiac cycles T1 plus a maximum broadcast interval T2 plus a broadcast time T3. Further, as can be seen from Fig. 3, since the implantable leadless pacemakers need to monitor the availability of the communication channel before the broadcast signals are transmitted, the second number is greater than or equal to the aforementioned first number.

It should be noted that the human heart rhythm follows a regular pattern (e.g., typically, right atrial pacing is followed by right ventricular pacing, which is in turn followed by left ventricular pacing). Accordingly, continuing the example of Fig. 3, in the implantable leadless pacing system including one programmer, three implantable leadless pacemakers, the beginning time of transmission of the first one of second broadcast information is the beginning time of transmission of the second broadcast information by the implantable leadless pacemaker Ca implanted in the right atrium, and the beginning time of transmission of the last one of second broadcast information is the beginning time of transmission of the second broadcast information by the implantable leadless pacemaker Cc implanted in the left ventricle. That is, the time occupied by the last one of second broadcast information is the time occupied by the second broadcast information transmitted by the implantable leadless pacemaker Cc implanted in the left ventricle. In another example, an implantable leadless pacing system includes a programmer, an implantable leadless pacemaker implanted in the right atrium and another implantable leadless pacemaker implanted in the right ventricle. In this implantable leadless pacing system, the beginning time of transmission of the first one of second broadcast information may be the beginning time of transmission of the second broadcast information transmitted by the implantable leadless pacemaker implanted in the right atrium, and the beginning time of transmission of the last one of second broadcast information may be the beginning time of transmission of the second broadcast information transmitted by the implantable leadless pacemaker implanted in the right ventricle. The channel time occupied by the last one of second broadcast information is the channel time occupied by the second broadcast information transmitted by the implantable leadless pacemaker implanted in the right ventricle.

### Embodiment 3

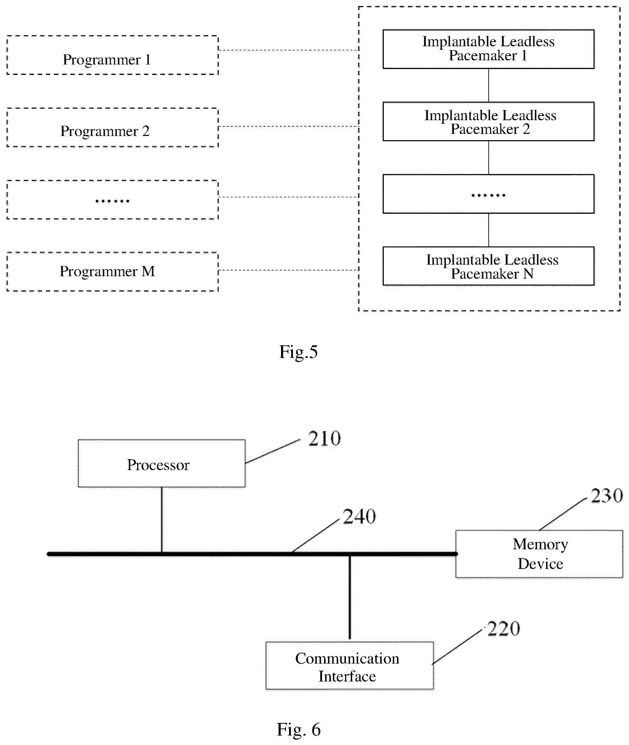

In a third embodiment, there is provided an implantable leadless pacing system. Specifically, reference is made to Fig. 5, a schematic diagram showing the structure of the implantable leadless pacing system of this embodiment. As can be seen from Fig. 5, in the present embodiment, the implantable leadless pacing system includes two implantable leadless pacemakers and a programmer. However, it should be noted that the present invention is not limited to any particular number of implantable leadless pacemakers, or to any particular locations where they are implanted in a patient's body. Optionally, in some implementations, the implantable leadless pacing system may include at least two implantable leadless pacemakers. In some other implementations, the implantable leadless pacing system may include at least one implantable leadless pacemaker and at least one programmer. In the case of the implantable leadless pacing system including four implantable leadless pacemakers, in some implementations, the four implantable leadless pacemakers may be implanted respectively in the right atrium, the left atrium, the right ventricle and the left ventricle of a patient. In some other implementations, two of the implantable leadless pacemakers may be implanted in a patient's right atrium, one of the implantable leadless pacemakers in the patient's right ventricle and the remaining implantable leadless pacemaker in the patient's left ventricle. In yet some other implementations, two of the implantable leadless pacemakers may be implanted in a patient's right atrium and the other two implantable leadless pacemakers in the patient's left atrium. Other implementations not enumerated herein are also possible. In practical use, an appropriate number of implantable leadless pacemakers may be implanted at appropriate locations according to the actual pacing requirements. In addition, the present invention is also not limited to any particular number of programmers in the implantable leadless pacing system. In some implementations, there may be no programmer in the implantable leadless pacing system. In some other implementations, there may be one programmer in the implantable leadless pacing system. In yet some other implementations, there may be two or more programmers in the implantable leadless pacing system. Each programmer may communicate data with one or more implantable leadless pacemakers according to a communication method described above in connection with the second embodiment.

More specifically, each implantable leadless pacemaker may communicate data with another implantable leadless pacemaker and a programmer according to a communication method described above in connection with the first embodiment, and each programmer may communicate data with an implantable leadless pacemaker according to the communication method of any implementation of the above-discussed second embodiment.

It should be noted that, and as would be appreciated by those skilled in the art, the implantable leadless pacing system may further include body surface ECG patch(es) (not shown) electrically connected to the programmer(s), which act(s) as a physical communication medium for data transmission between the programmer(s) and the implantable leadless pacemaker(s).

The implantable leadless pacing system of the present embodiment is based on the same inventive concept as the communication methods of the above-discussed first and second embodiments and therefore has at least all the advantages thereof. Further detailed description of those advantages is not repeated herein for the sake of brevity.

### Embodiment 4

In a fourth embodiment, there is provided an electronic device. Specifically, reference is made to Fig. 6, a schematic block diagram showing the structure of the electronic device of this embodiment. As shown in Fig. 6, the electronic device includes a processor 210 and a memory device 230 storing therein a computer program which, executed by the processor 210, implements a communication method as defined above. In particular, the electronic device of the present embodiment is based on the same inventive concept as the communication methods of the above-discussed first and second embodiments and therefore has at least all the advantages thereof. Further detailed description of those advantages is not repeated herein for the sake of brevity.

Specifically, without limitation, the electronic device may include an implantable leadless pacemaker or programmer of an implantable leadless pacing system as defined above.

More specifically, as shown in Fig. 6, the electronic device further includes a communication interface 220 and a communication bus 240. Communication among the processor 210, the communication interface 220 and the memory device 230 is accomplished through the communication bus 240. The communication bus 240 may be, among others, a peripheral component interconnect (PCI) bus or an extended industry standard architecture (EISA) bus. The communication bus 240 may be an address bus, data bus, control bus or other bus. Although it is represented by only one bold line in the figure for ease of illustration, this does not mean that there is only one bus, or only one type of bus. The communication interface 220 is used for communication of the electronic device with other devices.

The processor 210 suitable for use in this invention may be a central processing unit (CPU) or other general-purpose processor, a digital signal processor (DSP), an application specific integrated circuit (ASIC), a field-programmable gate array (FPGA) or other programmable logic device, a discrete gate or transistor logic device, a discrete hardware component, or the like. The general-purpose processor may be a microprocessor or any other conventional processor. The processor 210 may serve as a control center of the electronic device, and the various components in the electronic device may be interconnected with various interfaces and lines.

The memory device 230 may be used to store the computer program. The processor 210 can perform various functions of the electronic device by running or executing the computer program stored in the memory device 230 and retrieving data stored in the memory device 230.

The memory device 230 may include non-volatile and/or volatile memory. Non-volatile memory may include read-only memory (ROM), programmable ROM (PROM), electrically programmable ROM (EPROM), electrically erasable programmable ROM (EEPROM) or flash memory. Volatile memory may include random-access memory (RAM) or external cache memory. For illustration rather than limitation, RAM is available in many forms such as static RAM (SRAM), dynamic RAM (DRAM), synchronous DRAM (SDRAM), double-data-rate SDRAM (DDRSDRAM), enhanced SDRAM (ESDRAM), Synchlink DRAM (SLDRAM), Rambus direct RAM (RDRAM), Direct Rambus DRAM (DRDRAM), Rambus DRAM(RDRAM), etc.

### Embodiment 5

In a fifth embodiment, there is also provided a readable storage medium storing therein a computer program which, when executed by a processor, implements a communication method as described above in connection with the first or second embodiment.

Those skilled in the art will understand that the readable storage medium of the present embodiment is based on the same inventive concept as the communication methods of the above-discussed first and second embodiments and therefore has at least all the advantages thereof. Further detailed description of those advantages is not repeated herein for the sake of brevity.

According to embodiments of the present invention, the readable storage medium may be implemented as one of various computer-readable media, or any combination thereof. The readable media may be a computer-readable signal medium or a computer-readable storage medium. The computer-readable storage medium may be, for example, but is not limited to, an electronic, magnetic, optical, electromagnetic, infrared, or semiconductor system, apparatus, or device, or any suitable combination of the foregoing. More specific examples (a non-exhaustive list) of the computer-readable storage medium would include the following: an electrical connection having one or more wires, a portable computer diskette, a hard disk, a random access memory (RAM), a read-only memory (ROM), an erasable programmable read-only memory (EPROM or Flash memory), an optical fiber, a portable compact disc read-only memory (CD-ROM), an optical storage device, a magnetic storage device, or any suitable combination of the foregoing. As used herein, the phase "computer-readable storage medium" may refer to any tangible medium that can contain, or store a program for use by or in connection with an instruction execution system, apparatus, or device.

The computer-readable signal medium may include a propagated data signal with computer-readable program code embodied therein, for example, in baseband or as part of a carrier wave. Such a propagated signal may take any of a variety of forms, including, but not limited to, electro-magnetic, optical, or any suitable combination thereof. The computer-readable signal medium may also be any computer-readable medium that is not a computer-readable storage medium and that can communicate, propagate, or transmit a program for use by or in connection with an instruction execution system, apparatus, or device.

Computer program code for carrying out operations of the present invention may be written in any combination of one or more programming languages, including an object-oriented programming language such as Java, Smalltalk, C++ or the like and conventional procedural programming languages, such as the "C" programming language or similar programming languages. The program code may execute entirely on the user's computer, partly on the user's computer, as a stand-alone software package, partly on the user's computer and partly on a remote computer or entirely on a remote computer or server. In the latter scenario, the remote computer may be connected to the user's computer through any type of network, including a local area network (LAN) or a wide area network (WAN), or the connection may be made to an external computer (for example, through the Internet using an Internet Service Provider).

The communication methods, implantable leadless pacing system, electronic device and storage medium of the present invention have the following advantages over the prior art.

The communication method provided by the present invention is based on a feature of multi-device and single channel, by utilizing the heartbeat cycle to achieve the communication among multiple devices in an implantable leadless pacing system, and configuring reception windows to enable multiple devices to communicate during their appropriate time intervals to prevent signal collisions. In this way, communication of a carriers at the same frequency among multiple devices in the same patient's body over the same communication channel can be achieved without increasing the design complexity of an implantable leadless pacemaker. In addition, in contrast to the conventional practice of using carriers in different frequency bands, which leads to high power consumption of implantable leadless pacemakers involved, this can significantly extend the service life of the implantable leadless pacemakers, thereby saving resources and reducing the adverse influence on the patient's body of otherwise necessary frequent replacement of the implantable leadless pacemakers. Furthermore, the communication methods are directly applicable to existing implantable leadless pacing systems without making any hardware modifications to their implantable leadless pacemakers or programmers, making them easy-to-implement and low-cost methods.

The implantable leadless pacing system, electronic device and storage medium of the present invention are based on the same inventive concept as the communication methods of the present invention and therefore have at least all the advantages thereof. Further detailed description of those advantages is not repeated herein for the sake of brevity.

It is noted that the devices and methods in the embodiments disclosed herein may also be implemented in other ways and the device embodiments described above are merely illustrative. For example, the flowcharts and block diagrams in the figures illustrate the architecture, functionality and operation of possible implementations of devices, methods, and computer program products according to various embodiments of the present invention. In this regard, each block in the flowcharts or block diagrams may represent a module, segment, or portion of instructions, the module, segment, or portion of instructions comprises one or more executable instructions for implementing the specified logical function(s). It is to be also noted that, in some alternative implementations, the functions noted in the blocks may occur out of the order noted in the figures. For example, two blocks shown in succession may, in fact, be executed substantially concurrently, or the blocks may sometimes be executed in the reverse order, depending upon the functionality involved. It is further noted that each block of the block diagrams and/or flowchart illustrations, and combinations of blocks in the block diagrams and/or flowchart illustrations, can be implemented by special purpose hardware-based systems that perform the specified functions or acts or carry out combinations of special purpose hardware and computer instructions.

Further, the various functional modules in the embodiments disclosed herein may be integrated into a discrete component, or provided as separate modules. Alternatively, two or more such modules may be integrated into a discrete component.

The description presented above is merely that of some preferred embodiments of the present invention and is not intended to limit the scope thereof in any sense. Any and all changes and modifications made by those of ordinary skill in the art in light of the above teachings fall within the scope as defined in the appended claims. Apparently, those skilled in the art can make various modifications and variations to the present invention without departing from the spirit and scope thereof. Accordingly, the invention is intended to embrace all such modifications and variations if they fall within the scope of the appended claims and equivalents thereof.

## Claims

1. A communication method, used for a communication among a plurality of devices in an implantable leadless pacing system, wherein the plurality of devices includes: at least two implantable leadless pacemakers; or at least one leadless pacemaker and at least one programmer, wherein the communication method is implemented by each implantable leadless pacemaker and comprises:
based on an acquired intracavitary electrocardiogram (IC-ECG) signal, obtaining, by the implantable leadless pacemaker, a pacing event corresponding to the IC-ECG signal;
if the pacing event is a pacing event corresponding to the implantable leadless pacemaker, opening a first receive window of the implantable leadless pacemaker;
waiting until broadcast information of any other device is not received within an opening duration of the latest first receive window and after completion of the pacing event, broadcasting first broadcast information by the implantable leadless pacemaker, wherein the first broadcast information contains identification information of the implantable leadless pacemaker and the pacing event.

2. The communication method according to claim 1, further comprising:
opening the first receive window for the implantable leadless pacemaker at beginning of each cardiac cycle.

3. The communication method according to claim 1, wherein after the pacing event is determined to be a pacing event corresponding to the implantable leadless pacemaker, further comprising:
if a type of the pacing event is determined to be an abnormal pacing, delivering a pacing pulse by the implantable leadless pacemaker to complete the pacing event.

4. The communication method according to claim 1, further comprising:
with a first predetermined period being taken as a cycle, after completion of all pacing events in at least one cardiac cycle, broadcasting second broadcast information by the implantable leadless pacemaker through:
opening a second receive window of the implantable leadless pacemaker;
waiting until broadcast information of any other device is not received within an opening duration of the latest second receive window, broadcasting the second broadcast information of the implantable leadless pacemaker,
wherein the second broadcast information contains the identification information of the implantable leadless pacemaker, and wherein the first predetermined period comprises a first number of cardiac cycles, where the first number is an integer greater than or equal to 1.

5. The communication method according to claim 4, further comprising:
after each implantable leadless pacemaker broadcasts the second broadcast information, opening a third receive window to receive a third broadcast information from the programmer, wherein the third broadcast information contains programming information from the programmer for the implantable leadless pacemaker.

6. The communication method according to claim 5, wherein opening duration(s) of the first receive window and/or the second receive window and/or the third receive window of the implantable leadless pacemaker is(are) pre-configured according to a location where the implantable leadless pacemaker is implanted, or set through the programming information from the programmer.

7. A communication method used for a communication among a plurality of devices in an implantable leadless pacing system, wherein the plurality of devices includes: at least two implantable leadless pacemakers; or at least one leadless pacemaker and at least one programmer, and wherein the communication method is implemented by each programmer and comprises:
opening, by a programmer, a fourth receive window before broadcasting third broadcast information; and
waiting until broadcast information of any other device is not received within an opening duration of the latest fourth receive window, broadcasting the third broadcast information.

8. The communication method of claim 7, wherein waiting until broadcast information of any other device is not received within the opening duration of the latest fourth receive window and then broadcasting the third broadcast information further comprises:
broadcasting the third broadcast information by the programmer after receiving second broadcast information of the implantable leadless pacemaker, wherein the third broadcast information further contains identification information of the implantable leadless pacemaker associated with the programming information.

9. The communication method of claim 7, further comprising:
determining a duration of the fourth receive window by:
taking a sum of durations of a second predetermined period, a maximum broadcast interval of the second broadcast information of the implantable leadless pacemaker(s) and a broadcast time of the second broadcast information of the implantable leadless pacemaker as the opening duration of the fourth receive window, wherein the second predetermined period comprises a second number of cardiac cycles, which is an integer greater than or equal to 1; wherein the maximum broadcast interval of the second broadcast information of the implantable leadless pacemaker(s) is a time interval between a beginning time of transmission of the first one of second broadcast information and an beginning time of transmission of the last one of second broadcast information; and wherein the broadcast time of the second broadcast information of the implantable leadless pacemaker is a channel occupancy duration of the last one of the second broadcast information.

10. An implantable leadless pacing system, comprising at least two implantable leadless pacemakers, or at least one leadless pacemaker and at least one programmer, wherein each implantable leadless pacemaker implements the communication method as defined in any one of claims 1 to 6 to perform data transmission with the other implantable leadless pacemaker and/or the programmer, and wherein the programmer implements the communication method as defined in any one of claims 7 to 9 to perform data transmission with the implantable leadless pacemaker.

11. An electronic device, comprising a processor and a memory device, wherein the memory device has a computer program stored thereon that, when executed by the processor, implements the communication method as defined in any one of claims 1 to 9.

12. A readable storage medium, storing thereon a computer program that, when executed by a processor, implements the communication method as defined in any one of claims 1 to 9.
